(19) **Europäisches Patentamt**
**European Patent Office**
**Office européen des brevets**

(11) **EP 1 402 871 B1**

(12) **EUROPEAN PATENT SPECIFICATION**

(45) Date of publication and mention
of the grant of the patent:
**18.02.2009 Bulletin 2009/08**

(51) Int Cl.:
*A61L 33/00* *(2006.01)* *A61J 1/00* *(2006.01)*

(21) Application number: **02738623.4**

(22) Date of filing: **05.06.2002**

(86) International application number:
**PCT/JP2002/005542**

(87) International publication number:
**WO 2002/098344 (12.12.2002 Gazette 2002/50)**

(54) **ANTITHROMBOTIC COMPOSITIONS AND MEDICAL INSTRUMENTS CONTAINING THE SAME**

ANTITHROMBOTISCHE ZUSAMMENSETZUNGEN UND DIESE ENTHALTENDE MEDIZINISCHE INSTRUMENTE

COMPOSITIONS ANTITHROMBOTIQUES ET INSTRUMENTS MEDICAUX CONTENANT LESDITES COMPOSITIONS

(84) Designated Contracting States:
**DE FR GB IT**

(30) Priority: **05.06.2001 JP 2001169978**
**07.11.2001 JP 2001342335**

(43) Date of publication of application:
**31.03.2004 Bulletin 2004/14**

(73) Proprietor: **Toyo Boseki Kabushiki Kaisha**
**Osaka-shi,**
**Osaka 530-8230 (JP)**

(72) Inventors:
• **KASHIWABARA, Susumu**
**Ohtsu-shi**
**Shiga 520-0292 (JP)**

• **TANAKA, Hidenori**
**Ohtsu-shi**
**Shiga 520-0292 (JP)**
• **SATO, Masaki**
**Osaka 530-8230 (JP)**

(74) Representative: **Weber, Thomas**
**Patentanwälte**
**von Kreisler-Selting-Werner,**
**Postfach 10 22 41**
**50462 Köln (DE)**

(56) References cited:
**EP-A- 1 120 123          WO-A- 92/00747**
**JP-A- 4 197 264          JP-A- 7 108 061**
**JP-A- 9 169 801          JP-A- 9 187 502**
**JP-A- 11 164 882         US-A- 5 069 899**

**Description**

**Technical Field**

The present invention relates to an antithrombotic composition. When applied to a blood-contacting surface of a medical device to be used in contact with the blood, the antithrombotic composition of the present invention can impart superior antithrombotic property to the medical device without impairing the intrinsic function of the medical device.

More particularly, the present invention relates to an antithrombotic composition that [1] can impart long-lasting superior antithrombotic property and antibiotic property to a medical device, and [2] can impart long-lasting superior antithrombotic property to a medical device intended for use under the conditions where the blood flows extremely fast and moves vigorously.

In addition, the present invention relates to a medical device comprising the above-mentioned antithrombotic composition.

**Background Art**

There has been conventionally developed and put to practical use a technique for imparting antithrombotic property to the surface of medical devices (e.g., circuit tube for blood, tube for sampling monitor, intraaortic balloon pump, blood pump for artificial heart, catheter for angiography, artificial lung, venous reserver, artificial kidney, artery filter and the like) that comes into contact with the blood, thereby preventing coagulation of the blood. What has been mainly investigated for the improved antithrombotic property of a medical device is a technique for imparting antithrombotic property by fixing heparin or a heparin derivative having an anticoagulation effect on the blood-contacting surface of a medical device by

As a method for introducing heparin or a heparin derivative into the blood-contacting surface of a medical device, a method comprising introducing various functional groups into heparin or a heparin derivative and fixing them via a covalent bond with the functional group introduced onto the substrate of the blood-contacting surface of a medical device can be mentioned. As such method, for example, JP-A-4-197264 discloses a method comprising covalent binding one of the terminal glycidyl groups of amino group of heparin with polyethyleneglycol diglycidyl ether and covalent binding the other terminal glycidyl group with the amino group introduced onto the substrate.

In addition, JP-A-58-147404 discloses a method comprising covalent binding a free terminal aldehyde group produced by reducing the molecular weight of heparin with an amino group on the substrate of the blood-contacting surface of a medical device, and stabilizing the covalent bond by reduction reaction.

However, these methods are associated with the problems in that a step for introducing a group capable of reaction with heparin or a heparin derivative onto the substrate of a medical device is necessary, and that heparin or a heparin derivative is bonded too strongly to the substrate threby losing its degree of freedom, and cannot exhibit anticoagulant activity sufficiently. In addition, since these methods require multiple steps, the medical device becomes highly costly, posing a big problem under the circumstances where health care costs are running up.

As another method for imparting antithrombotic property to a medical device, for example, a method comprising bonding an organic cation compound such as ammonium salt, phosphonium salt and the like with a negative charge (sulfonic acid group, aminosulfonic acid group) possessed by heparin or a heparin derivative to form a complex insoluble in water and soluble in an organic solvent, and coating a medical device with the complex. For example, JP-B-48-13341 discloses a method for imparting antithrombotic property, which comprises reacting heparin with a cationic surfactant to form a haparin complex insoluble in water and soluble in an organic solvent, preparing a solution in which the complex is dissolved in an organic solvent alone or together with a plastic, coating the blood-contacting surface of a medical device with the solution and then drying the surface.

In the above-mentioned JP-B-48-13341, ammonium salts such as alkyltrimethylammonium chloride, dilauryldimethylammonium chloride and the like are recited as cationic surfactants. However, complexes of such ammonium salts and heparin have been found to allow early dissolution of the complex upon exposure to blood, resulting in the disappearance of antithrombotic property in a short period of time.

JP-B-48-13341 discloses, as a means to solve this problem, a complex of benzyldimethylstearylammonium salt, which is a benzalkonium salt (ammonium salt having one long chain alkyl group, two methyl groups and one benzyl group), wherein the long chain alkyl group has 18 carbon atoms, and heparin (corresponding to Comparative Example 6 of the present application). This method was able to solve the problem of disappearance of antithrombotic property in a short time; which is found in conventional complexes, to some extent. However, the use of the above-mentioned complex does not solve the problem of imparting superior antithrombotic property over a sufficiently long term.

US-A-5,069,899 and WO-A-92/00747 disclose a composition comprising heparin-benzalkonium chloride and tridodecylmethylammonium chloride as separate components of an antithrombotic composition.

In addition, J. Biomater. Sci. Polymer Edn, vol. 6 describes an ionic complex (corresponding to Comparative Example 7 of the present application) of heparin and dioctadecyldimethylammonium bromide, for the purpose of imparting superior antithrombotic property to a medical device over a long term. The composition comprising the ionic complex shows extremely high hydrophobicity of organic cation compound (dioctadecyldimethylammonium bromide) bonded to heparin. As a result, it can retain heparin on the surface of a medical device for a long time on the one hand, but on the other hand, the antithrombotic property of heparin is markedly suppressed, and when coated on a medical device, thrombus problematically tends to occur in the blood retention part, steps and the like.

The problem in the ionic complexes of heparin - organic cation compound so far considered is that the antithrombotic property cannot be retained over a prolonged period when organic cationic compounds having too high a hydrophilicity (e.g., water-soluble ammonium salts such as benzalkonium salt and the like) are used, because heparin elutes out in a short period, and sufficient performance cannot be exhibited when organic cationic compounds having too high a hydrophobicity (e.g., dioctadecyldimethylammonium bromide) are used, because anticoagulant activity of heparin is suppressed.

The present inventors considered that these problems cannot have been solved till present because heparin or a heparin derivative and only one kind of ammonium salt are used for conventional ionic complexes. In the ionic complex of heparin or a heparin derivative and only one kind of ammonium salt, because the total number of carbons of four aliphatic groups (e.g., alkyl group) of ammonium salt and the structure thereof are the same, the ionic complex shows the property too deviated toward hydrophilicity or hydrophobicity. As a result, heparin or a heparin derivative elutes out in blood in a short period, or elution of heparin or a heparin derivative is markedly suppressed, and the expected antithrombotic property cannot be exerted in either case. In other words, for a medical device to exert superior antithrombotic property, the ionic complex needs to have a suitable balance between hydrophilicity and hydrophobicity.

To solve such problems, the present inventors tried to maintain the antithrombotic property necessary and sufficient for a medical device for a long term by forming an ionic complex of two kinds of ammonium salts having different total numbers of carbons of four aliphatic alkyl groups bonded with heparin or a heparin derivative, thereby balancing the hydrophilicity and hydrophobicity of the ionic complex. To be specific, the present inventors have developed an ionic complex (corresponding to Comparative Example 4 and 5 of the present application) of at least two ammonium salts (dimethyldidodecylammonium salt and an ammonium salt having 30 to 38 carbon atoms in total and comprising at least two alkyl groups having not less than 10 carbon atoms) and heparin or a heparin derivative, and found that this complex can solve the above-mentioned problems [Japanese Patent No. 3228409 (JP-A-11-164882)].

A coating composition comprising the ionic complex described in the above-mentioned Japanese Patent No. 3228409 shows extremely high antithrombotic property, as compared to conventional heparin coating materials, when coated on a medical device (catheter and the like) used under the mild conditions where blood flow rate is not very fast and the movement of blood is gentle. However, the above-mentioned ionic complex has been found to be still insufficient in antithrombotic property for use on medical devices (e.g., artificial organs such as implant type artificial heart, external assistant artificial heart and the like) increasingly used in recent years and intended for use in contact with blood for a long term under conditions where blood flows extremely fast and the movement is violent. In other words, when a coating using a composition comprising the above-mentioned ionic complex is exposed to a quick blood flow and vigorous movement, heparin is quickly washed away in blood, thus making it difficult to maintain superior antithrombotic property over a long term.

Accordingly, there is a demand for an antithrombotic composition capable of affording superior antithrombotic property for a long period, even when applied to a medical device intended for use under the above-mentioned severe conditions. Such medical device is used in the environment where thrombus is basically difficult to occur due to fast flow of blood. Thus, the antithrombotic property per se may be lower than that of the materials shown by the present inventors in the above-mentioned Japanese Patent No. 3228409, but antithrombotic property needs to be maintained longer than conventional.

For the medical device as mentioned above, various complications during use due to bacterial infections also poses a problem. Accordingly, an antithrombotic composition having antibiotic property in addition to the antithrombotic property is also desired.

**Disclosure of the Invention**

It is therefore an object of the present invention to provide an antithrombotic composition capable of imparting superior antithrombotic property to a medical device over a prolonged period.

An object of the present invention is to provide an antithrombotic composition capable of imparting antithrombotic composition capable of imparting long-lasting superior antithrombotic property and antibiotic property to a medical device.

Still another object of the present invention is to provide an antithrombotic composition capable of imparting long-term superior antithrombotic property to a medical device intended for use under the conditions where blood flows extremely fast and the movement thereof is violent.

With the purpose of achieving the above-mentioned objects, the present inventors have found that an antithrombotic composition comprising an ionic complex formed from an organic cation mixture and heparin or a heparin derivative, wherein the organic cation mixture is a mixture of at least two ammonium salts, is useful. To be precise, the present inventors have found that, in the above-mentioned antithrombotic composition, the use of a mixture of (a) a water-soluble ammonium salt and (b) a water-insoluble ammonium salt as an organic cation mixture can impart a medical device with superior antithrombotic property as well as antibiotic property over a prolonged period. In addition, the present inventors have found that, in the above-mentioned antithrombotic composition, the use of a mixture of specific ammonium salts (c) and (d) described in detail in the following, as an organic cation mixture, imparts superior antithrombotic property over a prolonged period to a medical device intended for use under the conditions where blood flows extremely fast and the movement thereof is violent.

Accordingly, the present invention provides the following.

An antithrombotic composition comprising an ionic complex formed from an organic cation mixture and heparin or a heparin derivative, wherein the organic cation mixture is a mixture of at least one kind of (a) water-soluble ammonium salt which is selected from a benzethonium salt and a benzalkonium salt, and at least one kind of (b) water-insoluble ammonium salt, which is an ammonium salt wherein four aliphatic groups are bonded, provided that said organic cation mixture is not a mixture of a didodecyldimethylammonium salt and at least one kind of an ammonium salt selected from ammonium salts having four aliphatic groups, wherein the four aliphatic groups have 30 to 38 carbon atoms in total and at least two of the four aliphatic groups are each an alkyl group having not less than 10 carbon atoms.

Preferred embodiments are apparent from the dependent claims.

The present invention also relates to a medical device, wherein the above-mentioned antithrombotic composition is applied to at least a part of a blood-contacting surface.

## DETAILED DESCRIPTION OF THE INVENTION

The ammonium salt to be used in the present invention is a salt with halogen, such as fluorine, chlorine, bromine, iodine and the like, preferably ammonium chloride or ammonium bromide.

In the present invention, by the "antithrombotic property" is meant a property that resists easy coagulation of blood and easy formation of thrombus, particularly when the blood comes into contact with the surface of a medical device and the like.

In the present invention, "an antithrombotic composition comprising an ionic complex" and "antithrombotic composition" may contain, besides an ionic complex formed from the above-mentioned organic cation mixture and heparin or a heparin derivative, an ionic complex formed from an organic cation mixture other than the above-mentioned and heparin or a heparin derivative, as well as a physiologically active substance such as antibiotic materials and the like, a polymer material to be a binder, and other additives, as long as any substantial effects are exerted on the property of the above-mentioned ionic complex.

The antithrombotic composition of the present invention [1] characteristically contains an ionic complex formed by binding an organic cation mixture of at least one kind of (a) water-soluble ammonium salt, and at least one kind of (b) water-insoluble ammonium salt with heparin or a heparin derivative. Such an antithrombotic composition can impart superior antithrombotic property to a medical device over a prolonged period, because (b) water-insoluble ammonium salt strongly retains heparin or a heparin derivative on the blood-contacting surface of a medical device and (a) water-soluble ammonium salt prevents deviation of the ionic complex toward hydrophobicity, thereby appropriately adjusting the release rate of heparin or a heparin derivative.

Moreover, the antithrombotic composition of the present invention prevents adhesion to and propagation of bacteria in the medical device because (a) water-soluble ammonium salt is slowly released in blood to exert its antibiotic property, whereby various complications caused by bacterial infection can be prevented.

As used herein, by the "antibiotic property" of the present invention is meant suppression of generation, growth and proliferation of bacteria (preferably reduction).

As the (a) water-soluble ammonium salt contained in the organic cation mixture used in the present invention, benzethonium salt and benzalkonium salt can be mentioned. For example, aqueous solutions of mixtures of decylbenzyldimethylammonium salt, dodecylbenzyldimethylammonium salt, tetradecylbenzyldimethylammonium salt, hexadecylbenzyldimethylammonium salt, octadecylbenzyldimethylammonium salt and $C_8$-$C_{18}$ alkylbenzyldimethylammonium salt (aqueous solution for disinfection, commercially available in the trade name of Osvan and the like), benzethonium salt and the like are preferable, and dodecylbenzyldimethylammonium bromide, benzethonium chloride and tetradecylbenzyldimethylammonium chloride are more preferable.

The amount of the above-mentioned (a) water-soluble ammonium salt to be used is generally 5 to 70 wt%, preferably 10 to 50 wt%, more preferably 15 to 30 wt%, of the total weight of the organic cation mixture [i.e., total of the weights of (a) water-soluble ammonium salt and (b) water-insoluble ammonium salt]. When the (a) water-soluble ammonium salt exceeds 70 wt% of the organic cation mixture, it is eluted out in a large amount in the early stage of use of the medical device, which is not preferable. When it is less than 3 wt%, the ionic complex is deviated toward hydrophobicity, making release of heparin difficult, which in turn unpreferably results in degraded antithrombotic property of the medical device and a failure to impart sufficient antibiotic property to the medical device.

As the (b) water-insoluble ammonium salt in the organic cation mixture used in the present invention, an ammonium salt wherein four aliphatic groups are bonded are mentioned. Specifically, an ammonium salt wherein two of the four aliphatic groups are each an aliphatic alkyl group having not less than 12 carbon atoms and an ammonium salt wherein three of the four aliphatic groups are each an aliphatic alkyl group having not less than 10 carbon atoms are preferable.

Here, by the "water-insoluble" in the present invention means that, when a mixture containing an ammonium salt at a concentration of 1% of pure water is stirred at 40°C, which is near the intracorporeal temperature, it does not become a transparent homogenous solution and the ammonium salt becomes an oil that separates from water or remains in a dispersed cloudy state.

As the "ammonium salt wherein two of the four aliphatic groups are each an aliphatic alkyl group having not less than 12 carbon atoms" in the present invention, for example, didodecyldimethylammonium salt, ditetradecyldimethylammonium salt, dihexadecyldimethylammonium salt, dioctadecyldimethylammonium salt, dieicosanyldimethylammonium salt, dimethyldipalmitylammonium salt, didocosanyldimethylammonium salt, ditetraeicosanyldimethylammonium salt and the like can be mentioned. Preferred are ditetradecyldimethylammonium salt, dihexadecyldimethylammonium salt, dimethyldipalmitylammonium salt and dioctadecyldimethylammonium salt, and more preferred are ditetradecyldimethylammonium bromide, dioctadecyldimethylammonium bromide and dimethyldipalmitylammonium bromide.

As the above-mentioned "an ammonium salt wherein three of the four aliphatic groups are each an aliphatic alkyl group having not less than 10 carbon atoms" in the present invention, for example, tridecylmethylammonium salt, tridodecylmethylammonium salt, tritetradecylmethylammonium salt, trihexadecylmethylammonium salt, trioctadecylammonium salt, trieicosanylmethylammonium salt and the like can be mentioned. Preferred are tridodecylmethylammonium salt and trihexadecylmethylammonium salt.

Heparin or a heparin derivative can be used for the ionic complex used in the present invention. As the heparin derivative, heparin sodium, heparin potassium, heparin lithium, heparin calcium, heparin zinc, low molecular weight heparin, epoxylated heparin and the like can be mentioned but not limited thereto.

As a material for the medical device substrate of the present invention, any material generally used can be used. For example, resins (e.g., polyvinyl chloride, polycarbonate, polyethylene terephthalate, polyethylene, polypropylene, polymethylpentene, thermoplastic polyetherpolyurethane, thermosetting polyurethane, silicone rubber having a cross-linked portion, such as polydimethylsiloxane and the like, polymethylmethacrylate, polyvinylidene fluoride, tetrafluoropolyethylene, polysulfone, polyethersulfone, polyacetal, polystyrene, ABS resin, mixtures of these resins and the like), metals (e.g., stainless, titanium, aluminum and the like), and the like can be mentioned but not limited thereto.

The antithrombotic composition of the present invention is applied on the blood-contacting surface of a medical device by, for example, the following method but not limited thereto.

## Method 1

First, an organic cation mixture and heparin or a heparin derivative are mixed by stirring in a solvent to give precipitates containing an ionic complex. The precipitates were recovered, washed and unreacted heparin or a heparin derivative and ammonium salt are removed. Then the residue is purified by a known method to give an ionic complex. This ionic complex is dissolved in an organic solvent to give a solution. This solution is applied to the blood-contacting surface of a medical device by the method to be mentioned below and the solvent is removed by drying, whereby the surface acquires optimized antithrombotic property.

In this method, as a method for applying the above-mentioned solution to the surface of a medical device, soaking methods, spray methods, brushing methods and the like can be mentioned, but not limited thereto.

The organic solvent to be used for this method is not particularly limited as long as it does not degrade antithrombotic property of the ionic complex and does not impair the substrate surface of a medical device as far as possible. Generally, methanol, ethanol, isopropyl alcohol, n-propyl alcohol, n-hexane, cyclohexane, tetrahydrofuran (hereinafter THF), 1,4-dioxane, cyclohexanone, N,N-dimethylformamide, N,N-dimethylacetamide, N-methylpyrrolidone and the like can be mentioned.

## Method 2

Alternatively, the antithrombotic composition of the present invention can be applied by a method comprising dissolving an organic cation mixture in a suitable organic solvent, coating the solution to a blood-contacting surface of a medical device, removing the organic solvent by drying, applying an aqueous solution of heparin or a heparin derivative to form an antithrombotic composition comprising an ionic complex on the blood-contacting surface, and then removing water by drying.

In this method, the organic solvent used may be those recited above under Method 1.

## Examples

The present invention is explained in detail in the following by referring to Examples.

### Example 1

Dodecylbenzyldimethylammonium bromide (20 parts by weight, manufactured by Tokyo Kasei Kogyo Co., Ltd.) and ditetradecyldimethylammonium bromide (60 parts by weight, manufactured by Tokyo Kasei Kogyo Co., Ltd.) were dissolved in methanol (150 parts by weight). After confirmation of complete dissolution, water was added to a solution of this organic ammonium in methanol to the ratio of [methanol solution:water]=[30:70 (weight ratio)]. Then, ammonium salt partly precipitated in the solution but the solution became homogenous by raising the temperature of the solution to 50°C.

Heparin (30 parts by weight) was dissolved in ion exchanged water (150 parts by weight). Then, methanol was added to this aqueous heparin solution to the ratio of [aqueous heparin solution:methanol]=[70:30 (weight ratio)]. Then, heparin partly precipitated in the solution but the solution became homogenous by raising the temperature of the solution to 70°C.

The above-mentioned ammonium salt solution was added dropwise to the obtained heparin solution with stirring. The reaction product immediately precipitated in the solution. The precipitates were recovered, sufficiently washed to remove unreacted heparin and ammonium salt. The residue was separated by centrifugation to remove water content and then lyophilized to give an ionic complex used in the present invention (Example 1).

### Example 2

Benzethonium chloride (15 parts by weight, manufactured by Aldrich Chemical Company) and.dioctadecyldimethylammonium bromide (72 parts by weight, manufactured by Tokyo Kasei Kogyo Co., Ltd.) were dissolved in methanol (150 parts by weight), and in the same manner as in Example 1, an ionic complex used in the present invention (Example 2) was obtained.

### Example 3

Tetradecylbenzyldimethylammonium chloride (20 parts by weight, manufactured by Tokyo Kasei Kogyo Co., Ltd.) and dimethyldipalmitylammonium bromide (40 parts by weight, manufactured by Tokyo Kasei Kogyo Co., Ltd.) were dissolved in methanol (150 parts by weight), and in the same manner as in Example 1, an ionic complex used in the present invention (Example 3) was obtained.

### Comparative Example 1

Tridodecylmethylammonium bromide (60 parts by weight, manufactured by Tokyo Kasei Kogyo Co., Ltd.) was dissolved in methanol (150 parts by weight), and in the same manner as in Example 1, an ionic complex (Comparative Example 1) was obtained.

### Comparative Example 2

Dioctadecyldimethylammonium bromide (65 parts by weight, manufactured by Tokyo Kasei Kogyo Co., Ltd.) was dissolved in methanol (150 parts by weight), and in the same manner as in Example 1, an ionic complex (Comparative Example 2) was obtained.

### Comparative Example 3

Dodecylbenzyldimethylammonium chloride (30 parts by weight, manufactured by Tokyo Kasei Kogyo Co., Ltd.) was dissolved in methanol (150 parts by weight), and in the same manner as in Example 1, an ionic complex (Comparative Example 3) was obtained.

**Test 1** Evaluation of antithrombotic property

The antithrombotic property of the compositions including ionic complexes obtained in Examples 1 to 3 and Comparative Examples 1 to 3 was evaluated by the following method.

The ionic complexes obtained in the above-mentioned Examples and Comparative Examples were each dissolved in THF to the concentration of 1.0 wt%. This solution was passed through the inside of a polyvinyl chloride tube having an inner diameter of 3 mm and dried to coat the tube with the ionic complex.

For the observation of antithrombotic property under the environment similar to the actual use, saline heated to 37°C was circulated inside the tube coated with the above-mentioned ionic complex with a roller pump, and after lapse of one day, a sample was taken. The obtained sample was filled with citric acid-added bovine blood and incubated at 37°C. Then, 1/40 N calcium chloride solution was added to the sample to allow coagulation of the blood. After 3 min, trisodium citrate was added to the sample to stop the coagulation of the blood. The coagulated blood (thrombus) was taken, lyophilized and weighed. The results are shown under the column of "antithrombotic property" in Table 1. Here, the values in the column of antithrombotic property" show the relative weight ratio of the thrombus produced inside the polyvinyl chloride tube, when the amount of thrombus produced inside the glass tube having the same diameter as the polyvinyl chloride tube used in this test was 1.

For comparison, the antithrombotic property of an uncoated vinyl chloride tube was also evaluated by a similar method.

## Table 1

| | | Ex. 1 | Ex. 2 | Ex. 3 | Comp. Ex. 1 | Comp. Ex. 2 | Comp. Ex. 3 | not coated |
|---|---|---|---|---|---|---|---|---|
| Number of carbons (carbons) of four aliphatic groups constituting ammonium salts (a) and (b)* | (a) | 1, 1, 12, Bz | Benze-thonium | 1, 1, 14, Bz | – | – | 1, 1, 12, Bz | |
| | (b) | 1, 1, 14, 14 | 1, 1, 18, 18 | 1, 1, 16, 16 | 1, 12, 12, 12 | 1, 1, 18, 18 | – | |
| Anti-thrombotic property (weight ratio)** | Day 0 of circula-tion | 0 | 0 | 0 | 18 | 15 | 0 | 79 |
| | Day 1 of circula-tion | 3 | 8 | 6 | 24 | 28 | 38 | 75 |

* In Table, Bz means benzyl.

** relative weight ratio of the thrombus produced inside the polyvinyl chloride tube, when the amount of thrombus produced inside the glass tube having the same diameter as the polyvinyl chloride tube used in this test was 1

**Test 2** Evaluation of antibiotic property

The antibiotic property of the compositions including ionic complexes obtained in Examples 1 to 3 and Comparative Examples 1 to 3 was evaluated by the following method. A series of operation in this test were all performed aseptically.

A pseudomonas aeruginosa liquid (hereinafter this bacterium liquid is to be referred to as a bacterium stock solution) adjusted to a concentration of about $1 \times 10^7$ cells/ml with a broth liquid 50-fold diluted with sterilized saline was prepared.

The number of bacteria in this bacterium stock solution was measured as follows. A $10^4$-fold diluted solution (100 μl) of a bacterium stock solution was sown on a normal agar plate and the colonies of Pseudomonas aeruginosa formed in 24 hrs were counted. Taking this number of colony as N, the concentration C of the bacterium stock solution is represented by:

$$C = 10^4 \times N/0.1 = 10^5 \times N \ (cells/ml)$$

This bacterium stock solution (100 μl) was diluted with a broth liquid (40-fold diluted with sterilized saline) to adjust the total amount to 40 ml (hereinafter this liquid is to be referred to as a stock solution for immersion). A vinyl chloride film (hereinafter to be referred to as film A) previously cut into 5 cm×5 cm and EOG sterilized was immersed in this stock solution for immersion and cultured at 37°C for 24 hrs. After culture, the stock solution for immersion was diluted $10^4$ by 10-fold series with sterilized saline (hereinafter to be abbreviated as $10^n$-fold diluted liquid). Each diluted liquid (100 μl) was sown on a normal agar medium and after 24 hrs, the number of bacteria was counted for plates having 30 to 300 colonies of Pseudomonas aeruginosa formed on a normal agar medium. That is, when the counted colonies are $N_n$ colonies, the number of bacteria $N_a$ after contact with 25 cm$^2$ of film A is determined by the following formula:

$$N_a = 40 \times 10^n \times N_n/0.1$$

The concentration of the bacterium stock solution before contact with film A was as shown in the aforementioned C, the amount of the used stock solution was 100 μl. Thus, the number of bacterium $N_b$ before contact with film A is represented by the following formula:

$$N_b = C \times 0.1 = 10^4 \times N$$

The changes in number $N_b \rightarrow N_a$ due to contact with 25 cm$^2$ film A in 40 ml of stock solution for immersion are shown in Table 2. The decreased number of bacteria due to the contact means exertion of the antibiotic property of ionic complex applied onto the surface of film A. Here, "N.D." means that bacterium was not at all observed.

Table 2

| | Ex. 1 | Ex. 2 | Ex. 3 | Comp. Ex. 1 | Comp. Ex. 2 | Comp. Ex. 3 |
|---|---|---|---|---|---|---|
| Antibiotic property ($\times 10^5$ cells/40 ml) | 6.880 →N. D. | 6.880 →N.D. | 6.880 →N.D. | 6.880 →11. 220 | 6.880 →7.761 | 6.880 →N.D. |

The compositions containing the ionic complexes of Examples had both superior antithrombotic property and high antibiotic property over a prolonged period. This is because, in the compositions containing the ionic complexes of Examples, (b) water-insoluble ammonium salt strongly retains heparin or a heparin derivative on the blood-contacting surface of a medical device and (a) water-soluble ammonium salt prevents deviation of the ionic complex toward hydrophobicity, thereby appropriately adjusting the release rate of heparin, which in turn results in exertion of superior antithrombotic property over a prolonged period and antibiotic property due to (a) water-soluble ammonium salt.

In contrast, none of the compositions containing the ionic complex of Comparative Example showed superior antithrombotic property and antibiotic property over a prolonged period.

**Industrial Applicability**

The composition of the present invention contains an ionic complex formed by heparin or a heparin derivative and an organic cation mixture, which is a mixture of at least two ammonium salts. Therefore, it can impart superior antithrombotic property to a medical device over a prolonged period.

Particularly, the composition of the present invention comprises an ionic complex of an organic cation mixture which is a mixture of at least two ammonium salts, i.e., at least one kind of (a) water-soluble ammonium salt and at least one kind of (b) water-insoluble ammonium salt, and heparin or a heparin derivative. Therefore, it can impart a medical device with superior antithrombotic property as well as antibiotic property over a prolonged period.

**Claims**

1. An antithrombotic composition comprising an ionic complex formed from an organic cation mixture and heparin or a heparin derivative, wherein the organic cation mixture is a mixture of at least one kind of (a) water-soluble ammonium salt which is selected from a benzethonium salt and a benzalkonium salt, and at least one kind of (b) water-insoluble ammonium salt, which is an ammonium salt wherein four aliphatic groups are bonded, provided that said organic cation mixture is not a mixture of a didodecyldimethylammonium salt and at least one kind of an ammonium salt selected from ammonium salts having four aliphatic groups, wherein the four aliphatic groups have 30 to 38 carbon atoms in total and at least two of the four aliphatic groups are each an alkyl group having not less than 10 carbon atoms.

2. The antithrombotic composition of claim 1, wherein two of the four aliphatic groups are each an alkyl group having not less than 12 carbon atoms.

3. The antithrombotic composition of claim 1, wherein three of the four aliphatic groups are each an alkyl group having not less than 10 carbon atoms.

4. A medical device comprising the antithrombotic composition of any of claims 1 to 3, which is applied to at least a part of a blood-contacting surface.

**Patentansprüche**

1. Antithrombotische Zusammensetzung, die einen ionischen Komplex umfasst, der aus einem Gemisch organischer Kationen und Heparin oder einem Heparinderivat gebildet ist, wobei das Gemisch organischer Kationen ein Gemisch ist von wenigstens einem Vertreter von (a) wasserlösliches Ammoniumsalz, das aus einem Benzethoniumsalz und einem Benzalkoniumsalz ausgewählt ist, und wenigstens einem Vertreter von (b) wasserunlösliches Ammoniumsalz, das ein Ammoniumsalz ist, bei dem vier aliphatische Gruppen an den Stickstoff gebunden sind, mit der Maßgabe, dass das Gemisch organischer Kationen kein Gemisch aus einem Didodecyldimethylammoniumsalz und wenigstens einem Vertreter eines Ammoniumsalzes ist, das aus Ammoniumsalzen mit vier aliphatischen Gruppen ausgewählt ist, wobei die vier aliphatischen Gruppen insgesamt 30 bis 38 Kohlenstoffatome haben und wenigstens zwei der vier aliphatischen Gruppen jeweils eine Alkylgruppe mit nicht weniger als 10 Kohlenstoffatomen sind.

2. Antithrombotische Zusammensetzung gemäß Anspruch 1, wobei zwei der vier aliphatischen Gruppen jeweils eine Alkylgruppe mit nicht weniger als 12 Kohlenstoffatomen sind.

3. Antithrombotische Zusammensetzung gemäß Anspruch 1, wobei drei der vier aliphatischen Gruppen jeweils eine Alkylgruppe mit nicht weniger als 10 Kohlenstoffatomen sind.

4. Medizinische Vorrichtung, die die antithrombotische Zusammensetzung gemäß einem der Ansprüche 1 bis 3 umfasst, die auf wenigstens einen Teil einer blutkontaktierenden Oberfläche aufgetragen ist.

**Revendications**

1. Composition antithrombotique comprenant un complexe ionique formé à partir d'un mélange de cations organiques et d'héparine ou d'un dérivé d'héparine, dans laquelle le mélange de cations organiques est un mélange d'au moins un type de (a) sel d'ammonium hydrosoluble qui est choisi parmi un sel de benzéthonium et un sel de benzalkonium, et d'au moins un type de (b) sel d'ammonium insoluble dans l'eau qui est un sel d'ammonium dans lequel quatre groupes aliphatiques sont liés, à condition que ledit mélange de cations organiques ne soit pas un mélange d'un sel de didodécyldiméthylammonium et d'au moins un type de sel d'ammonium choisi parmi des sels d'ammonium possédant quatre groupes aliphatiques, dans lesquels les quatre groupes aliphatiques possèdent 30 à 38 atomes de carbone au total et au moins deux des quatre groupes aliphatiques sont chacun un groupe alkyle ne possédant pas moins de 10 atomes de carbone.

2. Composition antithrombotique selon la revendication 1, dans laquelle deux des quatre groupes aliphatiques sont chacun un groupe alkyle ne possédant pas moins 12 atomes de carbone.

3. Composition antithrombotique selon la revendication 1, dans laquelle trois des quatre groupes aliphatiques sont chacun un groupe alkyle ne possédant pas moins de 10 atomes de carbone.

4. Dispositif médical comprenant la composition antithrombotique selon l'une quelconque des revendications 1 à 3, qui est appliqué sur au moins une partie d'une surface au contact du sang.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4197264 A
- JP 58147404 A
- JP 48013341 B
- US 5069899 A
- WO 9200747 A
- JP 3228409 B
- JP 11164882 A

### Non-patent literature cited in the description

- *J. Biomater. Sci. Polymer Edn,* vol. 6